# EUROPEAN PATENT SPECIFICATION

(11) **EP 4 138 585 B1**
(45) Date of publication and mention of the grant of the patent: **21.02.2024**
(21) Application number: 21724355.9
(22) Date of filing: 23.04.2021
(51) Int. Cl.: A24B 15/167, A61K 31/465

(54) **AEROSOLISABLE FORMULATION**
AEROSOLIERBARE ZUSAMMENSETZUNG
FORMULATION AÉROSOLISABLE

(30) Priority: 24.04.2020 GB 202006008
(43) Date of publication of application: 01.03.2023
(73) Proprietor: Nicoventures Trading Limited, London WC2R 3LA (GB)
(72) Inventor: BURTON, Andrew, London WC2R 3LA (GB); MCLACHLAN, George, London WC2R 3LA (GB); BRUTON, Connor, London WC2R 3LA (GB); AZZOPARDI, Anna, London WC2R 3LA (GB); AKANDE, Olatunde, London WC2R 3LA (GB); MCKEOWN, Victoria, London WC2R 3LA (GB); MORRIS, Jay, London WC2R 3LA (GB); AGUIAR, Paulo, London WC2R 3LA (GB); CLAYTON, Peter, London WC2R 3LA (GB); SANCHEZ PENA, Maria Montserrat, London WC2R 3LA (GB)
(74) Representative: D Young & Co LLP
(86) International application number: PCT/GB2021/050997
(87) International publication number: WO 2021/214491

(56) References cited:
- WO-A1-2017/070706
- WO-A1-2017/133870
- WO-A1-2019/204502
- CN-A- 106 770 809

## Description

### FIELD OF THE INVENTION

The present disclosure relates to an aerosolisable formulation, a process for forming an aerosol from the aerosolisable formulation, containers which contain the aerosolisable formulation, electronic aerosol provision systems such as electronic aerosol delivery systems (e.g. e-cigarettes) incorporating said formulation, a process for preparing an aerosolisable formulation and the use of an aerosol formed from the aerosolisable formulation.

### BACKGROUND TO THE INVENTION

Electronic aerosol delivery systems such as e-cigarettes generally contain a reservoir of liquid (e-liquid) which is to be turned into an aerosol, typically containing nicotine. When a user inhales on the device, a heater is activated to aerosolise or vaporise a small amount of liquid, which is inhaled by the user. Other methods of aerosolising e-liquids are known in the art, such as by using pressurised gas or mechanical atomisation to generate an aerosol. Depending on the constituents of the aerosolisable formulation that are to be provided to a user, it may be preferable to formulate the aerosolisable formulation in a certain way. For example, it may be preferable to prepare the aerosolisable formulation so as to produce an aerosol with a particular profile. It may also be preferable to prepare the aerosolisable formulation so as to ensure the aerosolisable formulation meets certain standards of quality, consistency and the like. Document WO2017/070706 A1 discloses an aerosolisable formulation comprising synthetic nicotine for use in vaping devices and vaping devices employing this formulation.

It would thus be desirable to provide an aerosolisable formulation that is formulated so as to be acceptable to a user.

### SUMMARY OF THE INVENTION

In one aspect there is provided an aerosolisable formulation comprising:
(i) one or more aerosol-forming agents; and
(ii) nicotine;
wherein the nicotine comprises (R)-nicotine in an amount of from about 1% to about 4.5% by weight of the total nicotine.

In a further embodiment there is provided a process for forming an aerosol, the process comprising aerosolising an aerosolisable formulation comprising:
(i) one or more aerosol-forming agents; and
(ii) nicotine;
wherein the nicotine comprises (R)-nicotine in an amount of from about 1% to about 4.5% by weight of the total nicotine.

In a further embodiment there is provided a contained aerosolisable formulation comprising:
(a) a container; and
(b) an aerosolisable formulation , comprising
   (i) one or more aerosol-forming agents; and
   (ii) nicotine;
   wherein the nicotine comprises (R)-nicotine in an amount of from about 1% to about 4.5% by weight of the total nicotine.

In a further embodiment there is provided an electronic aerosol provision system comprising:
(a) an aerosoliser for turning a formulation into an aerosol for inhalation by a user of the electronic aerosol provision system;
(b) a power supply for supplying power to the aerosoliser; and
(c) an aerosolisable formulation comprising
   (i) one or more aerosol-forming agents; and
   (ii) nicotine;
   wherein the nicotine comprises (R)-nicotine in an amount of from about 1% to about 4.5% by weight of the total nicotine.

In a further embodiment there is provided a process for preparing an aerosolisable formulation comprising
(i) one or more aerosol-forming agents; and
(ii) nicotine;

wherein the nicotine comprises (R)-nicotine in an amount of from about 1% to about 4.5% by weight of the total nicotine;
the process comprising the steps of:
   (a)providing the nicotine; and
   (b)contacting the one or more aerosol-forming agents with the nicotine.

The present invention further provides the use of (R)-nicotine for reducing irritation of an aerosol when delivered to a user's oral or nasal cavity, wherein the aerosol is formed by aerosolising an aerosolisable formulation comprising
(i) one or more aerosol-forming agents; and
(ii) nicotine;
wherein the nicotine comprises (R)-nicotine in an amount of from about 1% to about 4.5% by weight of the total nicotine.

### DETAILED DESCRIPTION

As discussed herein, the present invention provides an aerosolisable formulation comprising (i) one or more aerosol-forming agents, and (ii) nicotine; wherein the nicotine comprises (R)-nicotine in an amount of from about 1% to about 4.5% by weight of the total nicotine.

Nicotine is the most abundant alkaloid in tobacco, accounting for approximately 95% of the total alkaloid content in tobacco. In addition, there are several minor chemical components in tobacco, such as nicotine- N'-oxide (e.g., nicotine-1'-N-oxide), nicotyrine (e.g., β-Nicotyrine), cotinine, nornicotyrine, 2',3-bipyridyl, anabasine, N-methyl anatabine, N-methyl anabasine, anabasine, and anatabine.

The nicotine molecule contains a chirogenic centre at the C2'-position of the pyrrolidine moiety and exists as two enantiomers, (R)-nicotine and (S)-nicotine, which are the only stereochemical stable alkaloids derivable from tobacco.

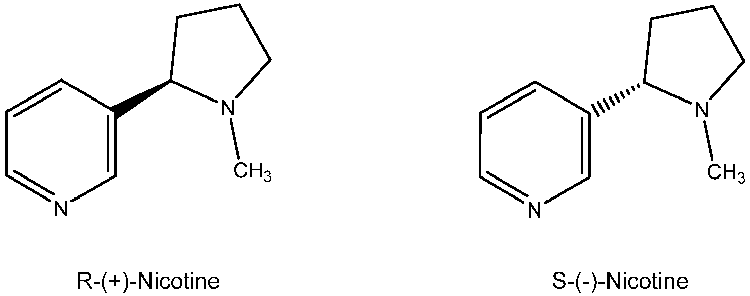

(S)-nicotine is reported to be the predominant naturally occurring enantiomeric form of nicotine in tobacco, whereas (R)-nicotine is a minor form of nicotine derived from tobacco, if present at all. Typically (R)-nicotine is present in naturally derived nicotine in an amount of less than 1% of total nicotine. Studies have shown that (R)-nicotine may be present in tobacco smoke in an amount of about 2% - 6% of the total nicotine content, which is likely the result of racemization during the combustion of tobacco.

Naturally derived (S)-nicotine is generally known to be more pharmacologically active than (R)-nicotine isomer, for example as a nicotinic acetylcholine receptor agonist.

We found that aerosolisable formulations comprising (R)-nicotine in an amount of from about 1% to about 4.5% by weight of the total nicotine content in the aerosolisable formulation provided an improved sensory experience and consumer satisfaction relative to aerosolisable formulations comprising predominantly (S)-nicotine. This was especially the case in aerosolisable formulations comprising high concentrations of nicotine.

We also found that aerosolisable formulations comprising (R)-nicotine in an amount of from about 1% to about 4.5% of the total nicotine content in the aerosolisable formulation enhanced the sensory effects associated with flavouring and additional active agents added to the aerosolisable formulation, thereby improving the sensory experience of the user inhaling the aerosol.

For ease of reference, these and further aspects of the present invention are now discussed under appropriate section headings. As will be understood by the skilled person, the teachings of each section are not limited to the section in question, but may be combined with other sections as appropriate.

### Nicotine

Nicotine may be provided in any suitable amount depending on the desired dosage when inhaled by the user. In one aspect nicotine is present in an amount of from about 0.1% to about 10% by weight of the aerosolisable formulation. In one aspect nicotine is present in an amount of from about 0.5% to about 10% by weight of the aerosolisable formulation. In one aspect nicotine is present in an amount of from about 1% to about 10% by weight of the aerosolisable formulation. In one aspect nicotine is present in an amount of from about 0.1% to about 9% by weight of the aerosolisable formulation. In one aspect nicotine is present in an amount of from about 0.5% to about 9% by weight of the aerosolisable formulation. In one aspect nicotine is present in an amount of from about 1% to about 9% by weight of the aerosolisable formulation. In one aspect nicotine is present in an amount of from about 0.1% to about 8% by weight of the aerosolisable formulation. In one aspect nicotine is present in an amount of from about 0.5% to about 8% by weight of the aerosolisable formulation. In one aspect nicotine is present in an amount of from about 1% to about 8% by weight of the aerosolisable formulation. In one aspect nicotine is present in an amount of from about 0.1% to about 7% by weight of the aerosolisable formulation. In one aspect nicotine is present in an amount of from about 0.5% to about 7% by weight of the aerosolisable formulation. In one aspect nicotine is present in an amount of from about 1% to about 7% by weight of the aerosolisable formulation. In one aspect nicotine is present in an amount of from about 0.1% to about 6% by weight of the aerosolisable formulation. In one aspect nicotine is present in an amount of from about 0.5% to about 6% by weight of the aerosolisable formulation. In one aspect nicotine is present in an amount of from about 1% to about 6% by weight of the aerosolisable formulation. In one aspect nicotine is present in an amount of from about 0.1% to about 5% by weight of the aerosolisable formulation. In one aspect nicotine is present in an amount of from about 0.5% to about 5% by weight of the aerosolisable formulation. In one aspect nicotine is present in an amount of from about 1% to about 5% by weight of the aerosolisable formulation. In one aspect nicotine is present in an amount of from about 0.1% to about 4% by weight of the aerosolisable formulation. In one aspect nicotine is present in an amount of from about 0.5% to about 4% by weight of the aerosolisable formulation. In one aspect nicotine is present in an amount of from about 1% to about 4% by weight of the aerosolisable formulation. In one aspect nicotine is present in an amount of from about 0.1% to about 3% by weight of the aerosolisable formulation. In one aspect nicotine is present in an amount of from about 0.5% to about 3% by weight of the aerosolisable formulation. In one aspect nicotine is present in an amount of from about 1% to about 3% by weight of the aerosolisable formulation. In one aspect nicotine is present in an amount of from about 0.1% to about 2.5% by weight of the aerosolisable formulation. In one aspect nicotine is present in an amount of from about 0.5% to about 2.5% by weight of the aerosolisable formulation. In one aspect nicotine is present in an amount of from about 1% to about 2.5% by weight of the aerosolisable formulation. In one aspect nicotine is present in an amount of from about 0.1% to about 2.0% by weight of the aerosolisable formulation. In one aspect nicotine is present in an amount of from about 0.5 to about 2.0% by weight of the aerosolisable formulation. In one aspect nicotine is present in an amount of from about 1% to about 2.0% by weight of the aerosolisable formulation. In one aspect nicotine is present in an amount of from about 0.1% to about 1.8% by weight of the aerosolisable formulation. In one aspect nicotine is present in an amount of from about 0.5% to about 1.8% by weight of the aerosolisable formulation. In one aspect nicotine is present in an amount of from about 1% to about 1.8% by weight of the aerosolisable formulation. In one aspect nicotine is present in an amount of from about 0.1% to about 1.5% by weight of the aerosolisable formulation. In one aspect nicotine is present in an amount of from about 0.5% to about 1.5% by weight of the aerosolisable formulation. In one aspect nicotine is present in an amount of from about 1% to about 1.5% by weight of the aerosolisable formulation. In one aspect nicotine is present in an amount of from about 0.1% to about 1.25% by weight of the aerosolisable formulation. In one aspect nicotine is present in an amount of from about 0.5% to about 1.25% by weight of the aerosolisable formulation. In one aspect nicotine is present in an amount of from about 1% to about 1.25% by weight of the aerosolisable formulation.

In one aspect nicotine is present in an amount of no greater than about 10% by weight of the aerosolisable formulation. In one aspect nicotine is present in an amount of no greater than about 9% by weight of the aerosolisable formulation. In one aspect nicotine is present in an amount of no greater than about 8% by weight of the aerosolisable formulation. In one aspect nicotine is present in an amount of no greater than about 7% based on the total weight of the aerosolisable formulation. In one aspect nicotine is present in an amount of no greater than about 6% by weight of the aerosolisable formulation. In one aspect nicotine is present in an amount of no greater than about 5% by weight of the aerosolisable formulation. In one aspect nicotine is present in an amount of no greater than about 4% by weight of the aerosolisable formulation. In one aspect nicotine is present in an amount of no greater than about 3% by weight of the aerosolisable formulation. In one aspect nicotine is present in an amount of no greater than about 2.5% by weight of the aerosolisable formulation. In one aspect nicotine is present in an amount of no greater than about 2.0% by weight of the aerosolisable formulation. In one aspect nicotine is present in an amount of no greater than about 1.8% by weight of the aerosolisable formulation. In one aspect nicotine is present in an amount of no greater than about 1.5% by weight of the aerosolisable formulation. In one aspect nicotine is present in an amount of no greater than about 1.25% by weight of the aerosolisable formulation. In one aspect nicotine is present in an amount of no greater than about 1.0% by weight of the aerosolisable formulation.

In one aspect nicotine is present in an amount of no greater than about 200mg/ml of the aerosolisable formulation. In one aspect nicotine is present in an amount of no greater than about 150mg/ml of the aerosolisable formulation. In one aspect nicotine is present in an amount of no greater than about 100mg/ml of the aerosolisable formulation. In one aspect nicotine is present in an amount of no greater than about 800mg/ml of the aerosolisable formulation. In one aspect nicotine is present in an amount of no greater than about 60mg/ml of the aerosolisable formulation. In one aspect nicotine is present in an amount of no greater than about 50mg/ml of the aerosolisable formulation. In one aspect nicotine is present in an amount of no greater than about 40mg/ml of the aerosolisable formulation. In one aspect nicotine is present in an amount of no greater than about 35mg/ml of the aerosolisable formulation. In one aspect nicotine is present in an amount of no greater than about 30mg/ml of the aerosolisable formulation. In one aspect nicotine is present in an amount of no greater than about 25mg/ml of the aerosolisable formulation. In one aspect nicotine is present in an amount of no greater than about 20mg/ml of the aerosolisable formulation. In one aspect nicotine is present in an amount of no greater than about 18mg/ml of the aerosolisable formulation. In one aspect nicotine is present in an amount of no greater than about 15mg/ml of the aerosolisable formulation. In one aspect nicotine is present in an amount of no greater than about 12mg/ml of the aerosolisable formulation. In one aspect nicotine is present in an amount of no greater than about 10mg/ml of the aerosolisable formulation. In one aspect nicotine is present in an amount of no greater than about 8mg/ml of the aerosolisable formulation. In one aspect nicotine is present in an amount of no greater than about 6mg/ml of the aerosolisable formulation. In one aspect nicotine is present in an amount of no greater than about 5mg/ml of the aerosolisable formulation. In one aspect nicotine is present in an amount of no greater than about 4mg/ml of the aerosolisable formulation. In one aspect nicotine is present in an amount of no greater than about 3mg/ml of the aerosolisable formulation. In one aspect nicotine is present in an amount of no greater than about 2mg/ml of the aerosolisable formulation. In one aspect nicotine is present in an amount of no greater than about 1mg/ml of the aerosolisable formulation.

In one aspect nicotine is present in an amount of at least about 1mg/ml of the aerosolisable formulation. In one aspect nicotine is present in an amount of at least about 2mg/ml of the aerosolisable formulation. In one aspect nicotine is present in an amount of at least about 3mg/ml of the aerosolisable formulation. In one aspect nicotine is present in an amount of at least about 4mg/ml of the aerosolisable formulation. In one aspect nicotine is present in an amount of at least about 5mg/ml of the aerosolisable formulation. In one aspect nicotine is present in an amount of at least about 6mg/ml of the aerosolisable formulation. In one aspect nicotine is present in an amount of at least about 8mg/ml of the aerosolisable formulation. In one aspect nicotine is present in an amount of at least about 10mg/ml of the aerosolisable formulation. In one aspect nicotine is present in an amount of at least about 12mg/ml of the aerosolisable formulation. In one aspect nicotine is present in an amount of at least about 15mg/ml of the aerosolisable formulation. In one aspect nicotine is present in an amount of at least about 18mg/ml of the aerosolisable formulation. In one aspect nicotine is present in an amount of at least about 20mg/ml of the aerosolisable formulation. In one aspect nicotine is present in an amount of at least about 25mg/ml of the aerosolisable formulation. In one aspect nicotine is present in an amount of at least about 30mg/ml of the aerosolisable formulation. In one aspect nicotine is present in an amount of at least about 35mg/ml of the aerosolisable formulation. In one aspect nicotine is present in an amount of at least about 40mg/ml of the aerosolisable formulation. In one aspect nicotine is present in an amount of at least about 50mg/ml of the aerosolisable formulation. In one aspect nicotine is present in an amount of at least about 60mg/ml of the aerosolisable formulation.

As discussed herein, nicotine may comprise (S)-nicotine or (R)-nicotine in any suitable amount depending on the desired effect on the aerosolisable formulation and provided (R)-nicotine is present in an amount of from about 1.0% to about 4.5% by weight of the total nicotine. In one aspect nicotine comprises (R)-nicotine in an amount of from about 1.0% to about 4.0% by weight of the total nicotine. In one aspect nicotine comprises (R)-nicotine in an amount of from about 1.0% to about 3.5% by weight of the total nicotine. In one aspect nicotine comprises (R)-nicotine in an amount of from about 1.0% to about 3.0% by weight of the total nicotine. In one aspect nicotine comprises (R)-nicotine in an amount of from about 1.5% to about 4.5% by weight of the total nicotine. In one aspect nicotine comprises (R)-nicotine in an amount of from about 1.5% to about 4.0% by weight of the total nicotine. In one aspect nicotine comprises (R)-nicotine in an amount of from about 1.5% to about 3.5% by weight of the total nicotine. In one aspect nicotine comprises (R)-nicotine in an amount of from about 1.5% to about 3.0% by weight of the total nicotine. In one aspect nicotine comprises (R)-nicotine in an amount of from about 2.0% to about 4.5% by weight of the total nicotine. In one aspect nicotine comprises (R)-nicotine in an amount of from about 2.0% to about 4.0% by weight of the total nicotine. In one aspect nicotine comprises (R)-nicotine in an amount of from about 2.0% to about 3.5% by weight of the total nicotine. In one aspect nicotine comprises (R)-nicotine in an amount of from about 2.0% to about 3.0% by weight of the total nicotine. In one aspect nicotine comprises (R)-nicotine in an amount of from about 2.5% to about 4.5% by weight of the total nicotine. In one aspect nicotine comprises (R)-nicotine in an amount of from about 2.5% to about 4.0% by weight of the total nicotine. In one aspect nicotine comprises (R)-nicotine in an amount of from about 2.5% to about 3.5% by weight of the total nicotine. In one aspect nicotine comprises (R)-nicotine in an amount of from about 2.5% to about 3.0% by weight of the total nicotine. In one aspect nicotine comprises (R)-nicotine in an amount of from about 3.0% to about 4.5% by weight of the total nicotine. In one aspect nicotine comprises (R)-nicotine in an amount of from about 3.0% to about 4.0% by weight of the total nicotine. In one aspect nicotine comprises (R)-nicotine in an amount of from about 3.0% to about 3.5% by weight of the total nicotine.

In one aspect nicotine is present in an amount of from about 0.1% to about 10% by weight of the aerosolisable formulation wherein nicotine comprises (R)-nicotine in an amount of from about 1.0% to about 4.0% by weight of the total nicotine. In one aspect nicotine is present in an amount of from about 0.1% to about 10% by weight of the aerosolisable formulation wherein nicotine comprises (R)-nicotine in an amount of from about 2.0% to about 4.0% by weight of the total nicotine. In one aspect nicotine is present in an amount of from about 0.1% to about 10% by weight of the aerosolisable formulation wherein nicotine comprises (R)-nicotine in an amount of from about 2.0% to about 3.0% by weight of the total nicotine. In one aspect nicotine is present in an amount of from about 0.1% to about 10% by weight of the aerosolisable formulation wherein nicotine comprises (R)-nicotine in an amount of from about 3.0% to about 4.0% by weight of the total nicotine.

In one aspect nicotine is present in an amount of from about 0.5% to about 5% by weight of the aerosolisable formulation wherein nicotine comprises (R)-nicotine in an amount of from about 1.0% to about 4.0% by weight of the total nicotine. In one aspect nicotine is present in an amount of from about 0.5% to about 5% by weight of the aerosolisable formulation wherein nicotine comprises (R)-nicotine in an amount of from about 2.0% to about 4.0% by weight of the total nicotine. In one aspect nicotine is present in an amount of from about 0.5% to about 5% by weight of the aerosolisable formulation wherein nicotine comprises (R)-nicotine in an amount of from about 2.0% to about 3.0% by weight of the total nicotine. In one aspect nicotine is present in an amount of from about 0.5% to about 5% by weight of the aerosolisable formulation wherein nicotine comprises (R)-nicotine in an amount of from about 3.0% to about 4.0% by weight of the total nicotine.

While it is know in the art that nicotine can be extracted and purified from naturally derived sources for use in aerosolisable formulations, nicotine may also be synthetically derived in order to provide the pure enantiomeric forms of nicotine. Naturally derived nicotine and synthetically derived nicotine are distinct and distinguishable from each for a number of reasons. The term "naturally derived" refer to materials that are produced from natural or organic sources, such as tobacco plants or leaves. The term "synthetically derived" refer to materials that are manufactured using synthetic chemical processes to provide the material. In other words, the term "synthetically derived" refer to materials that are manufactured using non-natural chemical processes to provide the material. Synthetically derived nicotine is chemically identical to naturally derived nicotine and therefore shares the same physiological properties.

While methods for preparing synthetic nicotine have been known since the 1940s, the high cost of production has limited its use in products intended for human consumption. However, new methods are being developed to improve efficiency and reduce the costs for preparing synthetic nicotine. Methods for preparing synthetically derived nicotine are described in WO 2017/117575 A1. For example, synthetic nicotine can be prepared using the following synthetic process:
1). React a nicotinate ester with N-vinyl-2-pyrrolidinone in the presence of a base and a solvent. Suitable bases may include metal hydrides comprising an alkali metal, such as lithium, potassium or sodium. Suitable solvents may include aromatic hydrocarbon or hydrocarbon solvents, dipolar aprotic solvents, ethers, polyethers, alcohols, toluene, xylenes, benzene and the like.
2). Combine mixture produced in step 1 with an acid, such as an aqueous hydrochloric acid solution, to form a second mixture comprising an aqueous layer.
3). Combine the separated aqueous layer with a concentrated acid, such as concentrated hydrochloric acid, to form a third mixture.
4). Combine the third mixture with a base to form a fourth mixture comprising myosamine, reducing myosamine to nornicotine using a reducing agent, such as a hydrogenation catalyst or a borohydride salt, and methylating the nornicotine to yield R, S-nicotine.

The above method produces a racemic mixture (50:50) of (R)- and (S)-nicotine with a purity that can exceed 99%.

In one aspect, the nicotine comprises a mixture of synthetically derived nicotine and naturally derived nicotine. In one aspect the nicotine comprises synthetically derived (R)-nicotine and naturally derived (S)-nicotine. In one aspect the nicotine comprises synthetically derived (R)- and (S)-nicotine. In one aspect the nicotine comprises synthetically derived (R)-nicotine and a mixture of synthetically derived (S)-nicotine and naturally derived (S)-nicotine.

In one aspect nicotine comprises synthetically derived (R)-nicotine in an amount of no greater than about 10% by weight of the total nicotine. In one aspect nicotine comprises synthetically derived (R)-nicotine in an amount of no greater than about 5% by weight of the total nicotine. In one aspect the nicotine comprises synthetically derived (R)-nicotine in an amount of no greater than about 3% by weight of the total nicotine.

In one aspect the nicotine comprises at least about 80% naturally derived (S)-nicotine, no greater than about 10% synthetically derived (S)-nicotine and no greater than about 10% synthetically derived (R)-nicotine by weight of the total nicotine. In one aspect the nicotine comprises at least about 90% naturally derived (S)-nicotine, no greater than about 5% synthetically derived (S)-nicotine and no greater than about 5% synthetically derived (R)-nicotine by weight of the total nicotine. In one aspect the nicotine comprises at least about 94% naturally derived (S)-nicotine, no greater than about 3% synthetically derived (S)-nicotine and no greater than about 3% synthetically derived (R)-nicotine by weight of the total nicotine.

In one aspect the nicotine comprises at least about 90% synthetically derived (S)-nicotine and no greater than about 10% synthetically derived (R)-nicotine by weight of the total nicotine. In one aspect the nicotine comprises at least about 95% synthetically derived (S)-nicotine and no greater than about 5% synthetically derived (R)-nicotine by weight of the total nicotine. In one aspect the nicotine comprises at least about 96% synthetically derived (S)-nicotine and no greater than about 4% synthetically derived (R)-nicotine by weight of the total nicotine. In one aspect the nicotine comprises at least about 97% synthetically derived (S)-nicotine and no greater than about 3% synthetically derived (R)-nicotine by weight of the total nicotine.

In one aspect, a racemic mixture of synthetically derived (R)- and (S)-nicotine can be combined with naturally derived nicotine, i.e. approximately 100% (S)-nicotine, in order to provide an aerosolisable formulation comprising nicotine and (R)-nicotine as defined in any one of the above embodiments.

### Aerosol-forming agents

The aerosolisable formulation of the present invention includes one or more aerosol-forming agents. An aerosol is a suspension of particles of liquid, solid, or both, within a gas. By the term "aerosol-forming agent" is meant a compound which when included in the formulation with the nicotine, and heated to a temperature between about 100 to about 250°C, forms a suspension of nicotine in its gaseous form. In other words, the aerosol-forming agent is involved in the formation of a suspension of nicotine upon heating of the formulation.

The one or more aerosol-forming agents can be selected from the group consisting of glycerin (also called glycerine or glycerol), 1,3-propanediol, 1,2-propanediol (propylene glycol) and sugar alcohols (e.g. sorbitol, ethylene glycol, erythritol, threitol, arabitol, xylitol, ribitol, mannitol, galactitol, fucitol, iditol, inositol, volemitol, isomalt, maltitol, lactitol). In one aspect the one or more aerosol-forming agents are selected from the group consisting of glycerol, 1,2-propanediol, 1,3-propanediol, diethylene glycol, triethylene glycol, tetraethylene glycol, 1,3-butylene glycol, erythritol, meso-Erythritol, ethyl vanillate, ethyl laurate, diethyl suberate, triethyl citrate, triacetin, a diacetin mixture, benzyl benzoate, benzyl phenyl acetate, tributyrin, lauryl acetate, lauric acid, myristic acid, propylene carbonate, and combinations thereof. In one aspect the one or more aerosol-forming agents are selected from the group consisting of glycerol, 1,2-propanediol, and mixtures thereof.

In one aspect the aerosolisable formulation of the present invention includes two or more aerosol-forming agents.

In one aspect the one or more aerosol-forming agents are present in a total amount of at least about 50% by weight of the aerosolisable formulation. In one aspect the one or more aerosol-forming agents are present in a total amount of at least about 60% by weight of the aerosolisable formulation. In one aspect the one or more aerosol-forming agents are present in a total amount of at least about 70% by weight of the aerosolisable formulation.

In one aspect the one or more aerosol-forming agents are present in a total amount of from about 50% to about 90% by weight of the aerosolisable formulation. In one aspect the one or more aerosol-forming agents are present in a total amount of from about 60% to about 90% by weight of the aerosolisable formulation. In one aspect the one or more aerosol-forming agents are present in a total amount of from about 70% to about 90% by weight of the aerosolisable formulation. In one aspect the one or more aerosol-forming agents are present in a total amount of from about 50% to about 80% by weight of the aerosolisable formulation. In one aspect the one or more aerosol-forming agents are present in a total amount of from about 60% to about 80% by weight of the aerosolisable formulation. In one aspect the one or more aerosol-forming agents are present in a total amount of from about 70% to about 80% by weight of the aerosolisable formulation.

### Other Components

The aerosolisable formulation of the present invention may include one or more further components. These components may be selected depending on the nature of the formulation.

In one aspect the aerosolisable formulation further comprises water. The water can be distinguished from the aerosol-forming agent(s) by the concentration at which it is included in the formulation.

In one aspect water is present in an amount of from about 0.1% to about 30% by weight of the aerosolisable formulation. In one aspect water is present in an amount of from about 5% to about 30% by weight of the aerosolisable formulation. In one aspect water is present in an amount of from about 10% to about 30% by weight of the aerosolisable formulation. In one aspect water is present in an amount of from about 15% to about 30% by weight of the aerosolisable formulation.

In one aspect the aerosolisable formulation is in the form of a liquid. In one aspect the aerosolisable formulation is not in the form of a gel.

In one aspect the formulation further comprises an active agent in addition to nicotine. By "active agent" it is meant an agent that imparts a physiologically active material, i.e. a material intended to achieve or enhance a physiological response. The additional active agent may for example be selected from nutraceuticals, nootropics, psychoactives. The additional active agent may be naturally occurring or synthetically obtained. The additional active agent may comprise for example caffeine, taurine, theine, vitamins such as B6 or B12 or C, melatonin, cannabinoids, or constituents, derivatives, or combinations thereof. The additional active agent may comprise one or more constituents, derivatives or extracts of cannabis or another botanical.

In one aspect, the additional active agent comprises caffeine, melatonin or vitamin B12.

As discussed herein, the additional active agent may comprise or be derived from one or more botanicals or constituents, derivatives or extracts thereof. As used herein, the term "botanical" includes any material derived from plants including, but not limited to, extracts, leaves, bark, fibres, stems, roots, seeds, flowers, fruits, pollen, husk, shells or the like. Alternatively, the material may comprise an active compound naturally existing in a botanical, obtained synthetically. The material may be in the form of liquid, gas, solid, powder, dust, crushed particles, granules, pellets, shreds, strips, sheets, or the like. Example botanicals are eucalyptus, star anise, hemp, cocoa, cannabis, fennel, lemongrass, peppermint, spearmint, rooibos, chamomile, flax, ginger, ginkgo biloba, hazel, hibiscus, laurel, licorice (liquorice), matcha, mate, orange skin, papaya, rose, sage, tea such as green tea or black tea, thyme, clove, cinnamon, coffee, aniseed (anise), basil, bay leaves, cardamom, coriander, cumin, nutmeg, oregano, paprika, rosemary, saffron, lavender, lemon peel, mint, juniper, elderflower, vanilla, wintergreen, beefsteak plant, curcuma, turmeric, sandalwood, cilantro, bergamot, orange blossom, myrtle, cassis, valerian, pimento, mace, damien, marjoram, olive, lemon balm, lemon basil, chive, carvi, verbena, tarragon, geranium, mulberry, ginseng, theanine, theacrine, maca, ashwagandha, damiana, guarana, chlorophyll, baobab or any combination thereof. The mint may be chosen from the following mint varieties: Mentha Arventis, Mentha c.v.,Mentha niliaca, Mentha piperita, Mentha piperita citrata c.v.,Mentha piperita c.v, Mentha spicata crispa, Mentha cardifolia, Memtha longifolia, Mentha suaveolens variegata, Mentha pulegium, Mentha spicata c.v. and Mentha suaveolens

In one aspect, the additional active agent comprises or derived from one or more botanicals or constituents, derivatives or extracts thereof and the botanical is selected from eucalyptus, star anise, cocoa and hemp. In one aspect, the additional active agent comprises or is derived from one or more botanicals or constituents, derivatives or extracts thereof and the botanical is selected from rooibos and fennel.

### Flavouring Agents

The aerosolisable formulation of the present invention may further comprise one or more a flavouring agents. The terms "flavouring agent", "flavour" or "flavourant" refer to materials which, where local regulations permit, may be used to create a desired taste, aroma or other somatosensorial sensation in a product for adult consumers. They may include naturally occurring flavour materials, botanicals, extracts of botanicals, synthetically obtained materials, or combinations thereof (e.g., tobacco, cannabis, licorice (liquorice), hydrangea, eugenol, Japanese white bark magnolia leaf, chamomile, fenugreek, clove, maple, matcha, menthol, Japanese mint, aniseed (anise), cinnamon, turmeric, Indian spices, Asian spices, herb, wintergreen, cherry, berry, red berry, cranberry, peach, apple, orange, mango, clementine, lemon, lime, tropical fruit, papaya, rhubarb, grape, durian, dragon fruit, cucumber, blueberry, mulberry, citrus fruits, Drambuie, bourbon, scotch, whiskey, gin, tequila, rum, spearmint, peppermint, lavender, aloe vera, cardamom, celery, cascarilla, nutmeg, sandalwood, bergamot, geranium, khat, naswar, betel, shisha, pine, honey essence, rose oil, vanilla, lemon oil, orange oil, orange blossom, cherry blossom, cassia, caraway, cognac, jasmine, ylang-ylang, sage, fennel, wasabi, piment, ginger, coriander, coffee, hemp, a mint oil from any species of the genus Mentha, eucalyptus, star anise, cocoa, lemongrass, rooibos, flax, ginkgo biloba, hazel, hibiscus, laurel, mate, orange skin, rose, tea such as green tea or black tea, thyme, juniper, elderflower, basil, bay leaves, cumin, oregano, paprika, rosemary, saffron, lemon peel, mint, beefsteak plant, curcuma, cilantro, myrtle, cassis, valerian, pimento, mace, damien, marjoram, olive, lemon balm, lemon basil, chive, carvi, verbena, tarragon, limonene, thymol, camphene), flavour enhancers, bitterness receptor site blockers, sensorial receptor site activators or stimulators, sugars and/or sugar substitutes (e.g., sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, liquid such as an oil, solid such as a powder, or gas.

In one aspect, the flavour comprises menthol, spearmint and/or peppermint. In one aspect, the flavour comprises flavour components of cucumber, blueberry, citrus fruits and/or redberry. In one aspect, the flavour comprises eugenol. In one aspect, the flavour comprises flavour components extracted from tobacco. In one aspect, the flavour comprises flavour components extracted from cannabis.

In one aspect, the flavour may comprise a sensate, which is intended to achieve a somatosensorial sensation which are usually chemically induced and perceived by the stimulation of the fifth cranial nerve (trigeminal nerve), in addition to or in place of aroma or taste nerves, and these may include agents providing heating, cooling, tingling, numbing effect. A suitable heat effect agent may be, but is not limited to, vanillyl ethyl ether and a suitable cooling agent may be, but not limited to eucolyptol, WS-3.

The flavouring agent is present in any amount to deliver the desired flavour to the aerosolisable formulation. The amount of the flavouring agent is defined with respect to the total weight of the aerosolisable formulation. In one aspect the flavouring agent is food-grade. As will be understood by the skilled person, the term "food-grade" refers to materials which are non-toxic and safe for human consumption.

In one aspect the flavouring agent is present in an amount of no greater than about 10 wt.% based on the aerosolisable formulation. In one aspect the flavouring agent is present in an amount of no greater than about 5 wt.%. In one aspect the flavouring agent is present in an amount of no greater than about 2 wt.%. In one aspect the flavouring agent is present in an amount of no greater than about 1.0 wt.%. In one aspect the flavouring agent is present in an amount of no greater than about 0.75 wt.%. In one aspect the flavouring agent is present in an amount of no greater than about 0.5 wt.%.

In one aspect the flavouring agent is present in an amount of from about 0.01 to about 10 wt.% based on the aerosolisable formulation. In one aspect the flavouring agent is present in an amount of from about 0.01 to about 5 wt.% based on the aerosolisable formulation. In one aspect the flavouring agent is present in an amount of from about 0.01 to about 2 wt.% based on the aerosolisable formulation. In one aspect the flavouring agent is present in an amount of from about 0.01 to about 1.0 wt.% based on the aerosolisable formulation. In one aspect the flavouring agent is present in an amount of from about 0.01 to about 0.75 wt.% based on the aerosolisable formulation. In one aspect the flavouring agent is present in an amount of from about 0.01 to about 0.5 wt.% based on the aerosolisable formulation.

It will be understood by the skilled person that the flavouring agent may be a multicomponent flavouring agent or a single component flavouring agent. When the formulation includes more than one such flavourants, each can be included at the above defined amounts.

### Processes

As discussed herein, the present invention provides a process for forming an aerosol, the process comprising aerosolising an aerosolisable formulation as defined herein. In one aspect, the aerosol is formed by a process performed at a temperature of from about 100 °C to about 250 °C.

In another aspect is a process for forming an aerosol, the process comprising aerosolising an aerosolisable formulation as defined herein by a process performed using a pressurised gas or mechanical atomisation.

Also discussed herein, the present invention provides a process for preparing an aerosolisable formulation comprising (i) one or more aerosol-forming agents; and (ii) nicotine; wherein the nicotine comprises (R)-nicotine in an amount of from about 1% to about 4.5% by weight of the total nicotine; the process comprising the steps of (a) providing the nicotine; and (b) contacting the one or more aerosol-forming agents with the nicotine. In one aspect of this process, step (a) comprises combining a racemic mixture of synthetic nicotine with naturally derived nicotine such that the amount of (R)-nicotine is from about 1% to about 4.5% by weight of the total nicotine. In one aspect of this process, step (a) comprises combining a racemic mixture of synthetic nicotine with naturally derived nicotine such that the amount of (R)-nicotine is in an amount as defined in any one of the embodiments defined herein.

### Further Aspects

The aerosolisable formulation may be contained or delivered by any means. In one aspect the present invention provides a contained aerosolisable formulation comprising (a) a container; and (b) an aerosolisable formulation as defined herein.

The container may be any suitable container, for example to allow for the storage or delivery of the solution. In one aspect the container is configured for engagement with an electronic aerosol provision system. The container may be a bottle. The container may be configured to become in communication with an electronic aerosol provision system, such as in fluid communication, so that solution may be delivered to the electronic aerosol provision system. As described above, the present disclosure relates to container which may be used in an electronic aerosol provision system, such as an e-cigarette. Throughout the following description the term "e-cigarette" is used; however, this term may be used interchangeably with electronic aerosol provision system.

As discussed herein, the container of the present invention is typically provided for the delivery of aerosolisable formulation to or within an e-cigarette. The aerosolisable formulation may be held within an e-cigarette or may be sold as a separate container for subsequent use with or in an e-cigarette. It is well known in the art that aerosolisable formulations that are suitable for use in e-cigarettes are often referred to as "e-liquids". In one aspect the aerosolisable formulation of the present invention is an e-liquid. As understood by one skilled in the art, e-cigarettes may contain a unit known as a detachable cartomiser which typically comprises a reservoir of aerosolisable formulation, a wick material and a device for aerosolising the aerosolisable formulation. In some e-cigarettes, the cartomiser is part of a single-piece device and is not detachable. In one aspect the container is a cartomiser or is part of a cartomiser. In one aspect the container is not a cartomiser or part of a cartomiser and is a container, such as a tank, which may be used to deliver aerosolisable formulation to or within an e-cigarette.

In one aspect the container is part of an e-cigarette. Therefore in a further aspect the present invention provides an electronic aerosol provision system comprising: (a) an aerosoliser for aerosolising formulation for inhalation by a user of the electronic aerosol provision system; (b) a power supply for supplying power to the aerosoliser (c) an aerosolisable formulation as defined herein. In one aspect the power supply comprises a cell or battery for supplying power to the aerosoliser.

In addition to the aerosolisable formulation of the present invention and to systems such as containers and electronic aerosol provision systems containing the same, the present invention provides use of (R)-nicotine for reducing irritation of an aerosol when delivered to a user's oral or nasal cavity, wherein the aerosol is formed by aerosolising an aerosolisable formulation comprising (i) one or more aerosol-forming agents; and (ii) nicotine; wherein the nicotine comprises (R)-nicotine in an amount of from about 1% to about 4.5% by weight of the total nicotine.

### Examples

### Example 1

The satisfaction performance of aerosolisable formulations comprising naturally derived or synthetically derived nicotine was evaluated.

A sequential monadic study was carried out involving 114 participants, who were either users of factory-made cigarettes (FMC) or dualists (consumers of both FMC and e-cigarettes). The study group consisted of 50% males and 50% females.

Each participant consumed 2 separate aerosolisable formulations comprising (i) 18mg/ml of naturally derived nicotine and (ii) 18mg/ml of synthetically derived nicotine. Both formulations consisted of 100% (S)-nicotine after initial product testing and were flavoured with "rich tobacco" flavour. The participants used an ePen3 aerosol delivery device (produced by British American Tobacco) to consume each formulation.

This study was used to assess the satisfaction performance of aerosolisable formulations comprising either naturally derived nicotine or synthetically derived nicotine.

**Table 1**

| | **Sample A** | **Sample B** |
|---|---|---|
| | Naturally Derived Nicotine | Synthetically Derived Nicotine |
| **Flavour liking (Mean)** | 2.7 | 2.8 |
| **Mild Taste (Mean)** | 2.5 | 2.4 |
| **Smooth Taste (Mean)** | 2.7 | 2.6 |
| **Not Bitter Taste (Mean)** | 3.0 | 2.8 |
| **Mild Pepper Taste (Mean)** | 3.2 | 3.2 |

| | | |
|---|---|---|
| *Significance test at 90% confidence interval* | | |

Flavour Liking was measured on a scale of 1 to 5: 1 representing a "dislike of flavour" and 5 representing a "liking of the flavour".

Mild Taste was measured on a scale of 1 to 5: 1 representing "strong taste" and 5 representing "mild taste".

Smooth Taste was measured on a scale of 1 to 5: 1 representing a "harsh taste" and 5 representing a "smooth taste".

Not Bitter Taste was measured on a scale of 1 to 5: 1 representing "bitter" and 5 representing "not bitter".

Mild Pepper Taste was measured on a scale of 1 to 5: 1 representing "strong taste" and 5 representing "mild peppery taste".

**Table 2**

| | **Sample A** | **Sample B** |
|---|---|---|
| | Naturally Derived Nicotine | Synthetically Derived Nicotine |
| **Flavour Liking (Mean)** | 2.7 | 2.9 |
| **Flavour Pleasantness (Mean)** | 2.7 | 2.7 |
| **Taste Intensity/Amount % JR** | 46% | 46% |
| **Aroma During Usage % JR** | 62% | 67% |
| **End of Usage After Taste % JR** | 48% | 48% |

| | | |
|---|---|---|
| *Significance test at 90% confidence interval* | | |

% JR is used to determine intensity of specific sensory attributes relative to one's hypothetical satisfactory level, which can affect the acceptance of the overall product. Acceptance tests are used to identify the liking of a product and individual product attributes. % JR is also frequently referred to as a "Just Right" (JR) scale or "Just about Right" scale (JAR).

**Table 3**

| | **Sample A** | **Sample B** |
|---|---|---|
| | Naturally Derived Nicotine | Synthetically Derived Nicotine |
| **Satisfaction (Mean)** | 2.7 | 2.9 |
| **Overall rating - T3B** | 46% | 50% |

| | | |
|---|---|---|
| *Significance test at 90% confidence interval* | | |

Satisfaction of the participant was measured on a scale of 1 to 5: 1 representing "unsatisfied" and 5 representing "satisfied".

The overall rating by the participant was measured on a scale of 1 to 5: 1 representing "poor" and 5 representing "excellent".

T3B is defined as a "Top 3 Box" score, associated with the Box Score analytical method. This analytical method is used as a way of summarising the positive responses from a Likert scale (i.e. a psychometric scale) survey question by combining the highest 3 responses to create a rating.

No significant difference was observed between the use of synthetically derived nicotine and naturally derived nicotine in terms of taste, flavour or pleasantness. Sample A and Sample B are therefore comparable.

### Example 2

The satisfaction performance of aerosolisable formulation comprising different racemic ratios of (S)- and (R)-nicotine was evaluated.

A sequential monadic study was carried out involving 114 participants, who were either users of factory-made cigarettes (FMC) or dualists (consumers of both FMC and e-cigarettes). The study group consisted of 50% males and 50% females.

Each participant consumed 4 aerosolisable formulations comprising of 18mg/ml of isomeric nicotine and flavoured with "rich tobacco" flavour. Said formulations consisted of naturally derived (S)-nicotine and synthetically derived (R)-nicotine in racemic ratios as defined in Tables 4-8. The participants used an ePen3 aerosol delivery device (produced by British American Tobacco) to consume each formulation.

**Table 4**

| | **Sample A** | **Sample C** | **Sample D** | **Sample E** |
|---|---|---|---|---|
| | 100% (S)-nicotine | 97% (S)-nicotine | 95% (S)-nicotine | 90% (S)-nicotine |
| | | 3% (R)-nicotine | 5% (R)-nicotine | 10% (R)-nicotine |
| **Flavour Liking (Mean)** | 2.7 | 2.9 | 2.8 | 2.9 |
| **Mild Taste (Mean)** | 2.5 | 2.9 A * | 3.1 AE * | 2.7 |
| **Smooth Taste (Mean)** | 2.7 | 3.0 a *^{†}* | 3.0 a *^{†}* | 2.8 |
| **Not Bitter Taste (Mean)** | 3.0 | 3.3 e *^{†}* | 3.2 | 3 |
| **Mild Pepper Taste (Mean)** | 3.2 | 3.6 ADE * | 3.4 e *^{†}* | 3.1 |

| | | | | |
|---|---|---|---|---|
| *†Significance test at 90% confidence interval* **Significance test at 95% confidence interval* | | | | |

Flavour Liking was measured on a scale of 1 to 5: 1 representing a "dislike of flavour" and 5 representing a "liking of the flavour".

Mild Taste was measured on a scale of 1 to 5: 1 representing "strong taste" and 5 representing "mild taste".

Smooth Taste was measured on a scale of 1 to 5: 1 representing a "harsh taste" and 5 representing a "smooth taste".

Not Bitter Taste was measured on a scale of 1 to 5: 1 representing "bitter" and 5 representing "not bitter".

Mild Pepper Taste was measured on a scale of 1 to 5: 1 representing "strong taste" and 5 representing "mild peppery taste".

Sample C was less bitter, less peppery and had a milder taste relative to Sample A, Sample D and Sample E, which provided for a better overall experience.

**Table 5**

| | **Sample A** | **Sample C** | **Sample D** | **Sample E** |
|---|---|---|---|---|
| | 100% (S)-nicotine | 97% (S)-nicotine | 95% (S)-nicotine | 90% (S)-nicotine |
| | | 3% (R)-nicotine | 5% (R)-nicotine | 10% (R)-nicotine |
| **Likes - Smooth/Not Harsh** | 7% | 25% A * | 18% A * | 17% A * |
| **Likes - Not Too Stronq** | 1% | 15% AE * | 8% A * | 3% |
| **Dislike - Don't Get a Hit of Nicotine** | 3% | 11%A* | 10% A * | 5% |
| **Dislike - Unpleasant Aftertaste** | 16% | 11% | 8% | 18% D * |
| **Dislike - Artificial Flavour** | 4% | 2% | 4% | 9% |

| | | | | |
|---|---|---|---|---|
| **Significance test at 95% confidence interval* | | | | |

Sample C was less bitter, less peppery and had a milder taste relative to Sample A, Sample D and Sample E, which provided for a better overall experience.

**Table 6**

| | **Sample A** | **Sample C** | **Sample D** | **Sample E** |
|---|---|---|---|---|
| | 100% (S)-nicotine | 97% (S)-nicotine | 95% (S)-nicotine | 90% (S)-nicotine |
| | | 3% (R)-nicotine | 5% (R)-nicotine | 10% (R)-nicotine |
| **Flavour Liking (Mean)** | 2.7 | 2.9 | 2.8 | 2.9 |
| **Flavour Pleasantness (Mean)** | 2.7 | 2.9 | 3.1 | 2.8 |
| **Taste Intensity/ Amount % JR** | 46% | 63% a *^{†}* | 57% | 54% |
| **Aroma During Usage % JR** | 62% | 66% | 70% | 70% |
| **End of Usage After Taste % JR** | 48% | 59% | 61% A * | 53% |

| | | | | |
|---|---|---|---|---|
| *†Significance test at 90% confidence interval* **Significance test at 95% confidence interval* | | | | |

Flavour Liking was measured on a scale of 1 to 5: 1 representing a "dislike of flavour" and 5 representing a "liking of the flavour".

Flavour Pleasantness was measured on a scale of 1 to 5: 1 representing "unpleasant" and 5 representing "pleasant".

% JR is used to determine intensity of specific sensory attributes relative to one's hypothetical satisfactory level, which can affect the acceptance of the overall product. Acceptance tests are used to identify the liking of a product and individual product attributes. % JR is also frequently referred to as a "Just Right" (JR) scale or "Just about Right" scale (JAR).

Sample C provided optimal taste intensity and end of usage aftertaste. Flavour liking and pleasantness were comparable to Sample A, i.e. formulations consisting of naturally derived (S)-nicotine and similar to the taste of natural tobacco.

**Table 7**

| | **Sample A** | **Sample C** | **Sample D** | **Sample E** |
|---|---|---|---|---|
| | 100% (S)-nicotine | 97% (S)-nicotine | 95% (S)-nicotine | 90% (S)-nicotine |
| | | 3% (R)-nicotine | 5% (R)-nicotine | 10% (R)-nicotine |
| **Irritation Level (Mean)** | 2.3 | 2.1 | 2.3 | 2.4 c *^{†}* |
| **Coughed at First Puff (% Yes)** | 35% | 28% | 33% | 48% ACD * |
| **Throat Kick - % JR** | 49% | 56% | 56% | 54% |
| **Vapour Inhaled % JR** | 70% | 71% | 76% | 74% |
| **Chest Impact % JR** | 68% | 66% | 66% | 70% |
| **Smoothness % JR** | 50% | 61% a *^{†}* | 58% | 52% |

| | | | | |
|---|---|---|---|---|
| *†Significance test at 90% confidence interval* **Significance test at 95% confidence interval* | | | | |

Irritation Level was measured on a scale of 1 to 5: 1 representing a "low irritation" and 5 representing a "high irritation".

Sample C provided optimal smoothness when consumed.

Sample D caused higher levels of coughing and irritation in study participants.

**Table 8**

| | **Sample A** | **Sample C** | **Sample D** | **Sample E** |
|---|---|---|---|---|
| | 100% (S)-nicotine | 97% (S)-nicotine | 95% (S)-nicotine | 90% (S)-nicotine |
| | | 3% (R)-nicotine | 5% (R)-nicotine | 10% (R)-nicotine |
| **Satisfaction (Mean)** | 2.7 | 3.0 A * | 2.9 | 2.9 |
| **Overall rating - T3B** | 46% | 56% | 52% | 48% |

| | | | | |
|---|---|---|---|---|
| **Significance test at 95% confidence interval* | | | | |

Satisfaction of the participant was measured on a scale of 1 to 5: 1 representing "unsatisfied" and 5 representing "satisfied".

The overall rating by the participant was measured on a scale of 1 to 5: 1 representing "poor" and 5 representing "excellent".

T3B is defined as a "Top 3 Box" score, associated with the Box Score analytical method. This analytical method is used as a way of summarising the positive responses from a Likert scale (i.e. a psychometric scale) survey question by combining the highest 3 responses to create a rating.

The satisfaction of participants was significantly higher for Sample C than for formulations consisting of (S)-nicotine, i.e. Sample A.

Sample D showed a directional advantage relative to Sample A and Sample E, while Samples A and E were considered comparable in terms of satisfaction and overall rating.

## Claims

1. An aerosolisable formulation comprising
(i) one or more aerosol-forming agents; and
(ii) nicotine;
wherein the nicotine comprises (R)-nicotine in an amount of from about 1% to about 4.5% by weight of the total nicotine.

2. An aerosolisable formulation according to claim 1, wherein the nicotine comprises (R)-nicotine in an amount of from about 2% to about 4% by weight of the total nicotine; such as wherein the nicotine comprises (R)-nicotine in an amount of from about 2% to about 3% by weight of the total nicotine; or wherein the nicotine comprises (R)-nicotine in an amount of from about 3% to about 4% by weight of the total nicotine.

3. An aerosolisable formulation according to any one of claims 1 to 4, wherein the nicotine is present in an amount of from about 0.1% to about 10% by weight of the aerosolisable formulation; such as wherein the nicotine is present in an amount of from about 0.5% to about 5% by weight of the aerosolisable formulation.

4. An aerosolisable formulation according to any one of claims 1 to 6, wherein the nicotine is present in an amount of no greater than about 1.8% by weight of the aerosolisable formulation.

5. An aerosolisable formulation according to any one of claims 1 to 7, wherein the nicotine comprises a mixture of synthetically derived nicotine and naturally derived nicotine.

6. An aerosolisable formulation according to any one of claims 1 to 8, wherein the one or more aerosol-forming agents is selected from the group consisting of glycerol, 1,2-propanediol, 1,3-propanediol, diethylene glycol, triethylene glycol, tetraethylene glycol, 1,3-butylene glycol, erythritol, meso-Erythritol, ethyl vanillate, ethyl laurate, diethyl suberate, triethyl citrate, triacetin, a diacetin mixture, benzyl benzoate, benzyl phenyl acetate, tributyrin, lauryl acetate, lauric acid, myristic acid, propylene carbonate, and combinations thereof; such as wherein the one or more aerosol-forming agents is selected from the group consisting of glycerol, 1,2-propanediol, and mixtures thereof.

7. An aerosolisable formulation according to any one of claims 1 to 10, wherein the one or more aerosol-forming agents are present in a total amount of at least about 50% by weight of the aerosolisable formulation; such as wherein the one or more aerosol-forming agents are present in a total amount of from about 60% to about 80% by weight of the aerosolisable formulation.

8. An aerosolisable formulation according to any one of claims 1 to 12, wherein the aerosolisable formulation further comprises water; such as wherein the water is present in an amount of from about 0.1% to about 30% by weight of the aerosolisable formulation; such as wherein the water is present in an amount of from about 15% to about 30% by weight of the aerosolisable formulation .

9. An aerosolisable formulation according to any one of claims 1 to 15, wherein the aerosolisable formulation further comprises one or more flavouring agents; such as wherein the one or more flavouring agents is selected from the group consisting of (4-(para-)methoxyphenyl)-2-butanone, vanillin, γ-undecalactone, menthone, 5-propenyl guaethol, menthol, para-mentha-8-thiol-3-one and mixtures thereof.

10. A process for forming an aerosol, the process comprising aerosolising an aerosolisable formulation comprising
(i) one or more aerosol-forming agents; and
(ii) nicotine;
wherein the nicotine comprises (R)-nicotine in an amount of from about 1% to about 4.5% by weight of the total nicotine.

11. A process according to claim 18 or 19 wherein the aerosol is formed by a process performed at a temperature of from about 100 °C to about 250 °C.

12. A contained aerosolisable formulation comprising
(a) a container; and
(b) an aerosolisable formulation , comprising
(i) one or more aerosol-forming agents; and
(ii) nicotine;
wherein the nicotine comprises (R)-nicotine in an amount of from about 1% to about 4.5% by weight of the total nicotine.

13. An electronic aerosol provision system comprising:
(a) an aerosoliser for turning a formulation into an aerosol for inhalation by a user of the electronic aerosol provision system;
(b) a power supply for supplying power to the aerosoliser; and
(c) an aerosolisable formulation comprising
(i) one or more aerosol-forming agents; and
(ii) nicotine;
wherein the nicotine comprises (R)-nicotine in an amount of from about 1% to about 4.5% by weight of the total nicotine.

14. A process for preparing an aerosolisable formulation comprising
(i) one or more aerosol-forming agents; and
(ii) nicotine;
wherein the nicotine comprises (R)-nicotine in an amount of from about 1% to about 4.5% by weight of the total nicotine;
the process comprising the steps of
(a) providing the nicotine; and
(b) contacting the one or more aerosol-forming agents with the nicotine.

15. Use of (R)-nicotine for reducing irritation of an aerosol when delivered to a user's oral or nasal cavity, wherein the aerosol is formed by aerosolising an aerosolisable formulation comprising
(i) one or more aerosol-forming agents; and
(ii) nicotine;
wherein the nicotine comprises (R)-nicotine in an amount of from about 1% to about 4.5% by weight of the total nicotine.

## Patentansprüche

1. Aerosolisierbare Formulierung, umfassend
(i) ein oder mehrere aerosolbildende Mittel und
(ii) Nicotin,
wobei das Nicotin (R)-Nicotin in einer Menge von etwa 1 Gew.-% bis etwa 4,5 Gew.-% des gesamten Nicotins umfasst.

2. Aerosolisierbare Formulierung nach Anspruch 1, wobei das Nicotin (R)-Nicotin in einer Menge von etwa 2 Gew.-% bis etwa 4 Gew.-% des gesamten Nicotins umfasst, wobei das Nicotin (R)-Nicotin beispielsweise in einer Menge von etwa 2 Gew.-% bis etwa 3 Gew.-% des gesamten Nicotins umfasst oder wobei das Nicotin (R)-Nicotin beispielsweise in einer Menge von etwa 3 Gew.-% bis etwa 4 Gew.-% des gesamten Nicotins umfasst.

3. Aerosolisierbare Formulierung nach einem der Ansprüche 1 bis 4, wobei das Nicotin in einer Menge von etwa 0,1 Gew.-% bis etwa 10 Gew.-% der aerosolisierbaren Formulierung vorhanden ist, wobei das Nicotin beispielsweise in einer Menge von etwa 0,5 bis etwa 5 Gew.-% der aerosolisierbaren Formulierung vorhanden ist.

4. Aerosolisierbare Formulierung nach einem der Ansprüche 1 bis 6, wobei das Nicotin in einer Menge von nicht mehr als etwa 1,8 Gew.-% der aerosolisierbaren Formulierung vorhanden ist.

5. Aerosolisierbare Formulierung nach einem der Ansprüche 1 bis 7, wobei das Nicotin eine Mischung aus synthetisch gewonnenem Nicotin und natürlich gewonnenem Nicotin umfasst.

6. Aerosolisierbare Formulierung nach einem der Ansprüche 1 bis 8, wobei das eine oder die mehreren aerosolbildenden Mittel aus der aus Glycerin, 1,2-Propandiol, 1,3-Propandiol, Diethylenglykol, Triethylenglykol, Tetraethylenglykol, 1,3-Butylenglykol, Erythrit, meso-Erythrit, Ethylvanillat, Ethyllaurat, Diethylsuberat, Triethylcitrat, Triacetin, einer Diacetinmischung, Benzylbenzoat, Benzylphenylacetat, Tributyrin, Laurylacetat, Laurinsäure, Myristinsäure, Propylencarbonat und Kombinationen davon bestehenden Gruppe ausgewählt sind, wobei das eine oder die mehreren aerosolbildenden Mittel beispielsweise aus der aus Glycerin, 1,2-Propandiol und Mischungen davon bestehenden Gruppe ausgewählt sind.

7. Aerosolisierbare Formulierung nach einem der Ansprüche 1 bis 10, wobei das eine oder die mehreren aerosolbildenden Mittel in einer Gesamtmenge von mindestens etwa 50 Gew.-% der aerosolisierbaren Formulierung vorhanden sind, wobei das eine oder die mehreren aerosolbildenden Mittel beispielsweise in einer Gesamtmenge von etwa 60 Gew.-% bis etwa 80 Gew.-% der aerosolisierbaren Formulierung vorhanden sind.

8. Aerosolisierbare Formulierung nach einem der Ansprüche 1 bis 12, wobei die aerosolisierbare Formulierung weiterhin Wasser umfasst, wobei das Wasser beispielsweise in einer Menge von etwa 0,1 Gew.-% bis etwa 30 Gew.-% der aerosolisierbaren Formulierung vorhanden ist, wobei das Wasser beispielsweise in einer Menge von etwa 15 bis etwa 30 Gew.-% der aerosolisierbaren Formulierung vorhanden ist.

9. Aerosolisierbare Formulierung nach einem der Ansprüche 1 bis 15, wobei die aerosolisierbare Formulierung weiterhin einen oder mehrere Geschacksstoffe umfasst, wobei der eine oder die mehreren Geschmacksstoffe beispielsweise aus der aus (4-(para-)Methoxyphenyl)-2-butanon, Vanillin, γ-Undecalacton, Menthon, 5-Propenylguaethol, Menthol, para-Mentha-8-thiol-3-on und Mischungen davon bestehenden Gruppe ausgewählt sind.

10. Verfahren zur Bildung eines Aerosols, wobei das Verfahren das Aerosolisieren einer aerosolisierbaren Formulierung umfasst, die Folgendes umfasst:
(i) ein oder mehrere aerosolbildende Mittel und
(ii) Nicotin,
wobei das Nicotin (R)-Nicotin in einer Menge von etwa 1 Gew.-% bis etwa 4,5 Gew.-% des gesamten Nicotins umfasst.

11. Verfahren nach Anspruch 18 oder 19, wobei das Aerosol durch ein bei einer Temperatur von etwa 100 °C bis etwa 250 °C durchgeführtes Verfahren gebildet wird.

12. In einem Behälter befindliche aerosolisierbare Formulierung, umfassend:
(a) einen Behälter und
(b) eine aerosolisierbare Formulierung, umfassend:
(i) ein oder mehrere aerosolbildende Mittel und
(ii) Nicotin,
wobei das Nicotin (R)-Nicotin in einer Menge von etwa 1 Gew.-% bis etwa 4,5 Gew.-% des gesamten Nicotins umfasst.

13. Elektronisches Aerosolbereitstellungssystem, umfassend:
(a) einen Vernebler zur Umwandlung einer Formulierung in ein Aerosol zur Inhalation durch einen Benutzer des elektronischen Aerosolbereitstellungssystems,
(b) eine Stromversorgung zur Stromversorgung des Verneblers und
(c) eine aerosolisierbare Formulierung, umfassend:
(i) ein oder mehrere aerosolbildende Mittel und
(ii) Nicotin,
wobei das Nicotin (R)-Nicotin in einer Menge von etwa 1 Gew.-% bis etwa 4,5 Gew.-% des gesamten Nicotins umfasst.

14. Verfahren zur Herstellung einer aerosolisierbaren Formulierung, umfassend
(i) ein oder mehrere aerosolbildende Mittel und
(ii) Nicotin,
wobei das Nicotin (R)-Nicotin in einer Menge von etwa 1 Gew.-% bis etwa 4,5 Gew.-% des gesamten Nicotins umfasst, wobei das Verfahren die folgenden Schritte umfasst:
(a) die Bereitstellung des Nicotins und
(b) das Inkontaktbringen des einen oder der mehreren aerosolbildenden Mittel mit dem Nicotin.

15. Verwendung von (R)-Nicotin zur Verminderung der Reizwirkung eines Aerosols bei der Verabreichung an die Mund- oder Nasenhöhle eines Anwenders, wobei das Aerosol gebildet wird durch Aerosolisieren einer aerosolisierbare Formulierung, umfassend
(i) ein oder mehrere aerosolbildende Mittel und
(ii) Nicotin,
wobei das Nicotin (R)-Nicotin in einer Menge von etwa 1 Gew.-% bis etwa 4,5 Gew.-% des gesamten Nicotins umfasst.

## Revendications

1. Formulation aérosolisable comprenant
(i) un ou plusieurs agents de formation d'aérosol ; et
(ii) de la nicotine ;
dans laquelle la nicotine comprend de la (R)-nicotine en une quantité comprise entre 1 % et environ 4,5 % en poids de la nicotine totale.

2. Formulation aérosolisable selon la revendication 1, dans laquelle la nicotine comprend de la (R)-nicotine en une quantité d'environ 2 % à environ 4 % en poids de la nicotine totale ; tel que dans laquelle la nicotine comprend de la (R)-nicotine en une quantité d'environ 2 % à environ 3 % en poids de la nicotine totale ; ou dans laquelle la nicotine comprend de la (R)-nicotine en une quantité d'environ 3 % à environ 4 % en poids de la nicotine totale.

3. Formulation aérosolisable selon l'une quelconque des revendications 1 à 4, dans laquelle la nicotine est présente en une quantité allant d'environ 0,1 % à environ 10 % en poids de la formulation aérosolisable ; tel que dans laquelle la nicotine est présente en une quantité allant d'environ 0,5 % à environ 5 % en poids de la formulation aérosolisable.

4. Formulation aérosolisable selon l'une quelconque des revendications 1 à 6, dans laquelle la nicotine est présente en une quantité ne dépassant pas environ 1,8 % en poids de la formulation aérosolisable.

5. Formulation aérosolisable selon l'une quelconque des revendications 1 à 7, dans laquelle la nicotine comprend un mélange de nicotine synthétiquement dérivée et de nicotine d'origine naturelle.

6. Formulation aérosolisable selon l'une quelconque des revendications 1 à 8, dans laquelle l'agent ou les agents de formation d'aérosol sont sélectionnés dans le groupe constitué par glycérol, 1,2-propane diol, 1,3-propane diol, diéthylène glycol, triéthylène glycol, tétraéthylène glycol, 1,3-butylène glycol, érythritol, méso-érythritol, vanillate d'éthyle, laurate d'éthyle, subérate de diéthyle, citrate de triéthyle, triacétine, un mélange de diacétine, benzoate de benzyle, acétate de benzylphényle, tributyrine, acétate de lauryle, acide laurique, acide myristique, carbonate de propylène et leurs combinaisons ; tel que dans laquelle l'agent ou les agents de formation d'aérosol sont sélectionnés dans le groupe composé de glycérol, de 1,2-propane diol et de mélanges de ceux-ci.

7. Formulation aérosolisable selon l'une quelconque des revendications 1 à 10, dans laquelle l'agent ou les agents de formation d'aérosols sont présents en une quantité totale d'au moins 50 % en poids de la formulation aérosolisable ; tel que dans laquelle l'agent ou les agents de formation d'aérosol sont présents en quantité totale d'environ 60 % à environ 80 % en poids de la formulation aérosolisable.

8. Formulation aérosolisable selon l'une quelconque des revendications 1 à 12, dans laquelle la formulation aérosolisable comprend en outre de l'eau ; tel que dans laquelle l'eau est présente en une quantité d'environ 0,1 % à environ 30 % en poids de la formulation aérosolisable ; tel que dans laquelle l'eau est présente en une quantité d'environ 15 % à environ 30 % en poids de la formulation aérosolisable .

9. Formulation aérosolisable selon l'une quelconque des revendications 1 à 15, dans laquelle la formulation aérosolisable comprend en outre un ou plusieurs agents aromatisants ; tel que dans laquelle l'agent ou les agents aromatisants sont sélectionnés dans le groupe constitué par (4-(para-)méthoxyphényl)-2-butanone, vanilline, γ-undécalactone, menthone, 5-propényl guaéthol, menthol, para-mentha-8-thiol-3-one des mélanges correspondants.

10. Procédé pour la formation d'un aérosol, le procédé comprenant l'aérosolisation d'une formulation aérosolisable comprenant
(i) un ou plusieurs agents de formation d'aérosol ; et
(ii) de la nicotine ;
dans laquelle la nicotine comprend de la (R)-nicotine en une quantité comprise entre 1 % et environ 4,5 % en poids de la nicotine totale.

11. Procédé selon la revendication 18 ou 19, dans lequel l'aérosol est formé par un procédé effectué à une température allant d'environ 100 °C à environ 250 °C.

12. Formulation aérosolisable contenue comprenant
(a) un récipient ; et
(b) une formulation aérosolisable, comprenant
(i) un ou plusieurs agents de formation d'aérosol ; et
(ii) de la nicotine ;
dans laquelle la nicotine comprend de la (R)-nicotine en une quantité allant d'environ 1 % à environ 4,5 % en poids de la nicotine totale.

13. Système électronique de fourniture d'aérosol comprenant :
(a) un aérosolisateur pour transformer une formulation en un aérosol pour inhalation par un utilisateur du système électronique de fourniture d'aérosol ;
(b) une alimentation en énergie pour fournir de l'énergie à l'aérosolisateur ; et
(c) une formulation aérosolisable comprenant :
(i) un ou plusieurs agents de formation d'aérosol ; et
(ii) de la nicotine ;
dans laquelle la nicotine comprend de la (R)-nicotine en une quantité allant d'environ 1 % à environ 4,5 % en poids de la nicotine totale.

14. Procédé pour la préparation d'une formulation aérosolisable comprenant
(i) un ou plusieurs agents de formation d'aérosol ; et
(ii) de la nicotine ;
dans laquelle la nicotine comprend de la (R)-nicotine en une quantité allant d'environ 1 % à environ 4,5 % en poids de la nicotine totale ;
le procédé comprenant les étapes de
(a) fourniture de la nicotine ; et
(b) mise en contact de l'agent ou des agents de formation d'aérosol avec la nicotine.

15. Utilisation de (R)-nicotine pour réduire l'irritation d'un aérosol lorsqu'il est délivré dans la cavité buccale ou nasale d'un utilisateur, dans laquelle l'aérosol est formé par aérosolisation d'une formulation aérosol comprenant
(i) un ou plusieurs agents de formation d'aérosol ; et
(ii) de la nicotine ;
dans laquelle la nicotine comprend de la (R)-nicotine en une quantité allant d'environ 1 % à environ 4,5 % en poids de la nicotine totale.
